(19) Europäisches Patentamt
European Patent Office
Office européen des brevets

(11) Publication number: 0 134 568
B1

# (12) EUROPEAN PATENT SPECIFICATION

(45) Date of publication of patent specification: 23.11.88

(51) Int. Cl.$^4$: A 61 K 31/535 // C07D498/04

(21) Application number: 84109944.3

(22) Date of filing: 21.08.84

## ERRATUM

| DIE TEXTSTELLE : / TEXT PUBLISHED : / LE PASSAGE SUIVANT : | (SEITE, SPALTE, ZEILE) (PAGE, COLUMN, LINE) (PAGE, COLONNE, LIGNE) | | | LAUTET BERICHTIGT: / SHOULD READ : / DEVRAIT ETRE LU : |
|---|---|---|---|---|
| containing a stabilizer selected from the group consisting of glucose, fructose and maltose, and an alkali metal salt of a mineral acid or carboxylic acid in a weight ratio of stabilizer to antibiotic of 0.01:1 to 0.5:1. | 4 | | 15/17 | containing glucose, fructose, maltose or an alkali metal salt of a mineral acid or carboxylic acid as stabilizer in a weight ratio of stabilizer to antibiotic of 0.01:1 to 0.5:1. |
| comprises dissolving a stabilizer selected from the group consisting of glucose, fructose and maltose, and an alkali metal salt of a mineral acid or carboxylic acid in a weight ratio of stabilizer to | 4 | | 39/40 | comprises dissolving glucose, fructose, maltose, or an alkali metal salt of a mineral acid or carboxylic acid as stabilizer in a weight ratio of stabilizer to |

Tag der Entscheidung über die Berichtigung / Date of decision on rectification. / Date de décision portant sur modification: 18.01.90

Ausgabe- und Veröffentlichungstag: / Issue and publication date: / Date d'edition et de publication: 28.03.90

Patbl.Nr) / EPB no:) 90/13 / Bull. no:)

(19) Europäisches Patentamt
European Patent Office
Office européen des brevets

(11) Publication number: **0 134 568**
**B1**

(12) # EUROPEAN PATENT SPECIFICATION

(45) Date of publication of patent specification: **23.11.88**

(51) Int. Cl.⁴: **A 61 K 31/535** // C07D498/04

(21) Application number: **84109944.3**

(22) Date of filing: **21.08.84**

(54) **Stable antibacterial lyophilizates.**

(30) Priority: **22.08.83 JP 153938/83**

(43) Date of publication of application:
**20.03.85 Bulletin 85/12**

(45) Publication of the grant of the patent:
**23.11.88 Bulletin 88/47**

(84) Designated Contracting States:
**AT BE CH DE FR IT LI NL SE**

(56) References cited:
**DE-A-3 124 592**
**GB-A-2 132 198**
**US-A-3 993 753**
**US-A-4 180 571**

(73) Proprietor: **SHIONOGI & CO., LTD.**
**12, Dosho-machi 3-chome Higashi-ku**
**Osaka 541 (JP)**

(72) Inventor: **Shima, Kazuhiro**
**3-17, Jyohoku-cho**
**Yamatokoriyama-shi Nara (JP)**
Inventor: **Inoue, Masayoshi**
**2-3-6-304, Tagawa Yodogawa-ku**
**Osaka-shi Osaka (JP)**
Inventor: **Tsukada, Takayuki**
**19-1, Niyanishi Yamada**
**Itami-shi Hyogo (JP)**

(74) Representative: **Vossius & Partner**
**Siebertstrasse 4 P.O. Box 86 07 67**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 134 568 B1**

## Description

This invention relates to a lyophilized preparation of a 7β-difluoromethylthioacetamido-7α-methoxy-3-[1-(2-hydroxy-$C_{2-4}$-alkyl)-1H-tetrazol-5-yl]thiomethyl-1-dethia-1-oxa-3-cephem-4-carboxylic acid alkali metal salt (I):

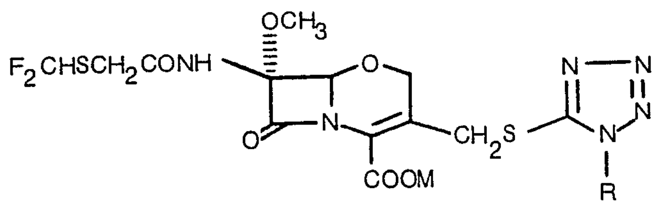

(I)

where M is an alkali metal cation and R is a $C_{2-4}$-β-hydroxyalkyl group containing at least one stabilizer or color preventing agent selected from glucose, fructose, maltose, and an alkali metal salt of a mineral acid or carboxylic acid as stabiliser in a weight ratio of stabilizer to antibiotic of 0.01:1 to 0.5:1.

In the formula (I), the preferred R group is the 2-hydroxyethyl and 2-hydroxypropyl group. M is preferably a sodium or potassium cation.

The compounds (I) (described in Japanese Patent Application No. 57—234,472) show a strong antibacterial potency against Gram-positive and -negative bacteria.

When the compounds are lyophilized conventionally for medical use, the product slowly develops a yellowish brown color and its antibacterial potency deteriorates.

The present inventors sought to overcome these defects and found that the said stabilizer or color preventing reagent is effective in an amount of 0.01 to 0.5 parts by weight per 1 part of the antibiotic.

It has been disclosed that sugars and sugar alcohols are effective for stabilizing lyophilized preparations of some beta-lactam antibiotics (Japanese Patent Kokai 57—11909).

Mannitol, the most effective in that case, is ineffective for stabilizing the present compounds (I). Only glucose, fructose, and maltose are effective sugars for stabilizing Compounds (I).

The alkali metal salt of a mineral acid is preferably that of hydrochloric acid, sulfuric acid, sulfurous acid or phosphoric acid, especially the non-toxic sodium or potassium salt. The alkali metal salt of a carboxylic acid is preferably an alkali metal salt of a $C_{3-6}$-dibasic aliphatic carboxylic acid, preferably succinic, maleic or fumaric acid, especially the non-toxic sodium or potassium salt.

The lyophilized preparation of this invention is produced as follows:

Compounds (I) and the said stabilizer in the weight ratio as stated above are dissolved in an aqueous solvent to give a solution. If required, the solution is adjusted to a pH of 5 to 8. The solution is cooled to freeze at −10 to −60°C over 2 minutes to 10 hours, and, if required under supplying sublimation heat, dried by sublimating water at a pressure of 0.005 to 1 millibar over 5 to 72 hours until the required water content is achieved. The product is preferably stored in a container, if required filled with an inert gas, e.g., nitrogen or dry air, and sealed.

Mechanical mixing of lyophilized Compound (I) and the said stabilizer does not stabilize the Compound (I).

This preparation is usually produced by conventional tray-, spray- or vial- lyophilization methods. The amount of said stabilizer is 0.01 to 0.5, especially 0.02 to 0.3 parts per 1 part by weight of Compound (I) to prevent the decomposition and color development. In the preparation, either Compound (I) or the stabilizer usually do not show an observable crystalline structure in its X-ray diffraction pattern.

The preparation produced in that way is highly soluble in water, easily produced sanitarily and ecologically, and is suitable for the preparation of compositions which can be injected. Further, it is suitable as a bulk material for long-term storage.

Said sterile preparation can be dissolved in a vehicle or solvent for injection before use and administered conventionally intravenously or intramuscularly.

Following examples illustrate embodiments of this invention.

### Example 1

A solution of Compound (I) (M=Na, R=—$CH_2CH_2OH$) (1 g) and stabilizer (glucose, fructose, or maltose) (0.3 g) in sterile distilled water for injection (4 ml) is poured into a 10 ml vial and cooled rapidly to −35°C in a refrigerator. After keeping at −35°C for 3 hours, the frozen mass is lyophilized at 0.1 millibar for 20 hours and 0.05 millibar or below for 6 hours at a temperature below −20°C to sublime the water and to afford a stable lyophilizate.

### Example 2

Substituting the stabilizer with 2 w/w % of sodium hydrogen sulfite, sodium sulfate, or sodium chloride, the method of Example 1 is repeated to give a stable preparation.

Example 3

Substituting the stabilizer with 1/4 millimole of sodium succinate, sodium fumarate, or sodium maleate, the method of Example 1 is repeated to afford a stable preparation.

Example 4

Substituting the amount of stabilizer by 0.1 g, the amount of sterile water by 10 ml, the volume of the vial by 100 ml, freezing by −40°C for 2 hours, and sublimation at 0.05 millibar for 10 hours and 0.005 millibar for 10 hours, the method of Example 1 is repeated to afford a stable preparation.

Example 5

Substituting the amount of stabilizer by 0.5 g, the method of Example 4 is repeated to afford a stable preparation.

TABLE

Stability of lyophilized preparation of Examples 1—3

| Stabilizer | Amount vs Ig of Compd. (I) | Potency (50°C, 1 month) |
|---|---|---|
| Glucose | 30 (w/w%) | 89.1 (%) |
| Fructose | " | 86.6 |
| Maltose | " | 89.3 |
| $NaHSO_3$ | 2 (w/w%) | 85.5 |
| $Na_2SO_4$ | " | 84.7 |
| NaCl | " | 86.8 |
| Na succinate | 0.25 (mM) | 86.0 |
| Na fumarate | " | 85.3 |
| Na maleate | " | 86.4 |
| Reference | — | 83.3 |

Example 6

Substituting the amount of stabilizer by 1 w/w % or 10 w/w %, freezing by −28°C for 5 hours, and sublimation at 0.5 millibar for 10 hours and 0.1 millibar for 20 hours, the method of Example 2 is repeated to afford a stable preparation.

Example 7

Substituting the amount of stabilizer by 5 w/w % or 8 w/w %, the amount of sterile water by 10 ml, the volume of the vial by 100 ml, freezing by −30°C for 4 hours 15 minutes, and sublimation at 0.2 millibar for 24 hours and 0.04 millibar for 8 hours, the method of Example 2 is repeated to afford a stable preparation.

Example 8

Substituting the amount of stabilizer by 1/2 millimoles or 1 millimole, the method of Example 3 is repeated to afford a stable preparation.

Example 9

Example 3 is repeated, however, Compound (I) — M=K,

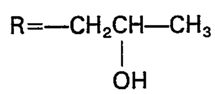

— the stabilizer, and sterile water are used in a ten-fold amount. The components are mixed and the pH of the resulting solution is adjusted to 6.7 with a phosphate buffer. The neutral solution is poured into a

lyophilization tray and lyophilized under the conditions of Example 3 to afford a stable preparation.

Lyophilizates of Examples 1 to 9 do not show the development of a yellowish brown color as observed in the case of a lyophilized material containing none of said stabilizers. In a test at 50°C, the stable lyophilizate did not develop a deeper color than pale yellow up to 3 months and retains its antibacterial potency up to 95% for up to 1 month.

Experiment

A solution of the sterile product of Examples 1 to 3 in distilled water for injection (4 ml) is administered by intravenous injection or drip to a patient suffering from a *Staphylococcus aureus* infection three times a day to treat the infection.

**Claims for the Contracting States: BE CH DE FR IT LI NL SE**

1. A lyophilized preparation of a 7β-difluoromethylthioacetamido-7α-methoxy-3-[1-(2-hydroxy-$C_{2-4}$-alkyl)-1H-tetrazol-5-yl]thiomethyl-1-dethia-1-oxa-3-cephem-4-carboxylic acid alkali metal salt, containing a stabilizer selected from the group consisting of glucose, fructose and maltose, and an alkali metal salt of a mineral acid or carboxylic acid in a weight ratio of stabilizer to antibiotic of 0.01:1 to 0.5:1.

2. A preparation as claimed in Claim 1, where the antibiotic is 7β-difluoromethylthioacetamido-7α-methoxy-3-[1-(2-hydroxyethyl)-1H-tetrazol-5-yl]thiomethyl-1-dethia-1-oxa-3-cephem-4-carboxylic acid sodium salt.

3. A preparation as claimed in Claim 1, where the stabilizer is an alkali metal salt of a mineral acid.

4. A preparation as claimed in Claim 1, where the stabilizer is sodium hydrogen sulfite, sodium sulfate or sodium chloride.

5. A preparation as claimed in Claim 1, where the stabilizer is a sodium salt of a $C_{3-6}$ dibasic carboxylic acid selected from succinic acid, fumaric acid, and maleic acid.

6. A preparation claimed in Claims 1 to 5, where the weight ratio of stabilizer to antibiotic is 0.02:1 to 0.3:1.

7. A process for preparing a lyophilized preparation claimed in Claim 1 which comprises dissolving the stabilizer and the antibiotic in a weight ratio of 0.01:1 to 0.5:1 in a sterile aqueous solvent, neutralizing the solution to pH 5 to 8, freezing at a temperature between −10 to −60°C for 2 minutes to 10 hours, and drying the frozen material under high vacuum between 0.005 and 1 millibar for 5 to 72 hours.

8. A process as claimed in Claim 7 which is carried out by vial, spray or tray lyophilization.

9. A process as claimed in Claim 7, where the lyophilized preparation is kept under dry nitrogen.

**Claims for the Contracting State: AT**

1. A process for preparing lyophilized preparation of a 7β-difluoromethylthioacetamido-7α-methoxy-3-[1-(2-hydroxy-$C_{2-4}$-alkyl)-1H-tetrazol-5-yl]thiomethyl-1-dethia-1-oxa-3-cephem-4-carboxylic acid alkali metal salt which comprises dissolving a stabilizer selected from the group consisting of glucose, fructose and maltose, and an alkali metal salt of a mineral acid or carboxylic acid in a weight ratio of stabilizer to antibiotic of 0.01:1 to 0.5:1 in a sterile aqueous solvent, neutralizing the solution to pH 5 to 8, freezing at a temperature between −10 to −60°C for 2 minutes to 10 hours, and drying the frozen material under high vacuum between 0.005 and 1 millibar for 5 to 72 hours.

2. A process as claimed in Claim 1, where the antibiotic is 7β-difluoromethylthioacetamido-7α-methoxy-3-[1-(2-hydroxyethyl)-1H-tetrazol-5-yl]thiomethyl-1-dethia-1-oxa-3-cephem-4-carboxylic acid sodium salt.

3. A process as claimed in Claim 1, where the stabilizer is an alkali metal salt of a mineral acid.

4. A process as claimed in Claim 1, where the stabilizer is sodium hydrogen sulfite, sodium sulfate, or sodium chloride.

5. A process as claimed in Claim 1, where the stabilizer is a sodium salt of a $C_{3-6}$ dibasic carboxylic acid selected from succinic acid, fumaric acid, and maleic acid.

6. A process as claimed in Claims 1 to 5, where the weight ratio of stabilizer to antibiotic is 0.02:1 to 0.3:1.

7. A process as claimed in Claim 1 which is carried out by vial, spray or tray lyophilization.

8. A process as claimed in Claim 7, where the lyophilized preparation is kept under dry nitrogen.

**Patentansprüche für die Vertragsstaaten: BE CH DE FR IT LI NL SE**

1. Gefriergetrocknetes Präparat eines Alkalimetallsalzes der 7β-Difluormethylthioacetamido-7α-methoxy-3-[1-(2-hydroxy-$C_{2-4}$-alkyl)-1H-tetrazol-5-yl]-thiomethyl-1-dethia-1-oxa-3-cephem-4-carbonsäure, enthaltend Glukose, Fructose, Maltose oder ein Alkalimetallsalz einer Mineral- oder Carbonsäure als Stabilisator in einem Gewichtsverhältnis von Stabilisator zu Antibiotikum von 0,01:1 bis 0,5:1.

2. Präparat nach Anspruch 1, in dem das Antibiotikum das Natriumsalz der 7β-Difluormethylthioacetamido-7α-methoxy-3-[1-(2-hydroxyäthyl)-1H-tetrazol-5-yl]-thiomethyl-1-dethia-1-oxa-3-cephem-4-carbonsäure ist.

3. Präparat nach Anspruch 1, in dem der Stabilisator ein Alkalimetallsalz einer Mineralsäure ist.

4. Präparat nach Anspruch 1, in dem der Stabilisator Natriumhydrogensulfit, Natriumsulfat oder Natriumchlorid ist.

5. Präparat nach Anspruch 1, in dem der Stabilisator ein Natriumsalz einer dibasischen $C_{3-6}$-Carbonsäure, wie Bernsteinsäure, Fumarsäure oder Maleinsäure, ist.

6. Präparat nach einem der Ansprüche 1 bis 5, in dem das Gewichtsverhältnis von Stabilisator zu Antibiotikum 0,02:1 bis 0,3:1 ist.

7. Verfahren zur Herstellung eines gefriergetrockneten Präparats nach Anspruch 1, bei dem der Stabilisator und das Antibiotikum in einem Gewichtsverhältnis von 0,01:1 bis 0,5:1 in einem sterilen wäßrigen Lösungsmittel gelöst werden, der pH-Wert der Lösung auf 5 bis 8 neutralisiert wird, die Lösung 2 Minuten bis 10 Stunden bei einer Temperatur zwischen −10 bis −60°C eingefroren wird und bei dem das gefrorene Material 5 bis 72 Stunden bei stark vermindertem Druck zwischen 0,005 und 1 Millibar getrocknet wird.

8. Verfahren nach Anspruch 7, bei dem eine Ampullen-, Sprüh oder Schalengefriertrockung erfolgt.

9. Verfahren nach Anspruch 7, bei dem das gefriergetrocknete Präparat unter trockener Stickstoffatmosphäre gehalten wird.

**Patentansprüche für den Vertragsstaat: AT**

1. Verfahren zur Herstellung eines gefriergetrockneten Präparates eines Alkalimetallsalzes der 7β-Difluormethylthioacetamido-7α-methoxy-3-[1-(2-hydroxy-$C_{2-4}$-alkyl)-1H-tetrazol-5-yl]-thiomethyl-1-dethia-1-oxa-3-cephem-4-carbonsäure, bei dem man Glukose, Fructose, Maltose oder ein Alkalimetallsalz einer Mineral- oder Carbonsäure als Stabilisator in einem gewichtsverhältnis von Stabilisator zu Antibiotikum von 0,01:1 bis 0,5:1 in einem sterilen wäßrigen Lösungsmittel löst, den pH-Wert der Lösung auf 5 bis 8 neutralisiert, 2 Minuten bis 10 Stunden bei einer Temperatur zwischen −10 bis −60°C einfriert und das gefrorene Material 5 bis 72 Stunden unter stark vermindertem Druck zwischen 0,005 und 1 Millibar trocknet.

2. Verfahren nach Anspruch 1, bei dem das Antibiotikum das Natriumsalz der 7β-Difluormethylthioacetamido-7α-methoxy-3-[1-(2-hydroxyäthyl)-1H-tetrazol-5-yl]-thiomethyl-1-dethia-1-oxa-3-cephem-4-carbonsäure ist.

3. Verfahren nach Anspruch 1, bei dem der Stabilisator ein Alkalimetallsalz einer Mineralsäure ist.

4. Verfahren nach Anspruch 1, in dem der Stabilisator ein Natriumhydrogensulfit, Natriumsulfat oder Natriumchlorid ist.

5. Verfahren nach Anspruch 1, bei dem der Stabilisator ein Natriumsalz einer dibasischen $C_{3-6}$-Carbonsäure, wie Bernsteinsäure, Fumarsäure oder Maleinsäure, ist.

6. Verfahren nach Anspruch 1 bis 5, bei dem das Gewichtsverhältnis von Stabilisator zu Antibiotikum 0,02:1 bis 0,3:1 ist.

7. Verfahren nach Anspruch 1, bei dem eine Ampullen-, Sprüh- oder Schalengefriertrocknung erfolgt.

8. Verfahren nach Anspruch 7, bei dem das gefriergetrocknete Präparat unter trockener Stickstoffatmosphäre gehalten wird.

**Revendications pour les Etats contractants: BE CH DE FR IT LI NL SE**

1. Préparation lyophilisée d'un sel de métal alcalin d'acide 7β-difluorométhylthioacétamido-7α-méthoxy-3-[1-(2-hydroxy-$C_{2-4}$-alkyl)-1H-tétrazol-5-yl]thiométhyl-1-déthia-1-oxa-3-céphen-4-carboxylique, contenant de glucose, du fructose, du maltose ou du sel de métal alcalin d'un acide minéral ou d'un acide carboxylique comme stabilisant dans un rapport en poids du stabilisant à l'antibiotique de 0,01:1 à 0,5:1.

2. Préparation suivant la revendication 1, caractérisée en ce que l'antibiotique est le sel de sodium d'acide 7β-difluorométhylthioacétamido-7α-méthoxy-3-[1-(2-hydroxyéthyl)-1H-tétrazol-5-yl]thiométhyl-1-déthia-1-oxa-3-céphen-4-carboxylique.

3. Préparation suivant la revendication 1, caractérisée en ce que le stabilisant est un sel de métal alcalin d'un acide minéral.

4. Préparation suivant la revendication 1, caractérisée en ce que le stabilisant est du bisulfite de sodium, du sulfate de sodium ou du chlorure de sodium.

5. Préparation suivant la revendication 1, caractérisée en ce que le stabilisant est un sel de sodium d'un acide carboxylique dibasique en $C_{3-6}$ choisi parmi l'acide succinique, l'acide fumarique et l'acide maléique.

6. Préparation suivant l'une quelconque des revendications 1 à 5, caractérisée en ce que le rapport en poids du stabilisant à l'antibiotique est de 0,02:1 à 0,3:1.

7. Procédé de préparation d'une préparation lyophilisée suivant la revendication 1, qui comprend la dissolution du stabilisant et de l'antibiotique dans un rapport en poids de 0,01:1 à 0,5:1 dans un solvant aqueux stérile, la neutralisation de la solution à pH 5—8, la congélation à une température se situant entre −10 et −60°C pendant 2 minutes à 10 heures, et le séchage de la matière congelée sous un vide élevé entre 0,005 et 1 millibar pendant 5 à 72 heures.

8. Procédé suivant la revendication 7, caractérisé en ce qu'il est réalisé au moyen d'une lyophilisation en flacon, par vaporisation ou en plateau.

9. Procédé suivant la revendication 7, caractérisé en ce que la préparation lyophilisée maintenue sous de l'azote sec.

**Revendications pour l'Etat contractant: AT**

1. Procédé de préparation d'une préparation lyophilisée d'un sel de métal d'acide 7β-difluorométhylthioacétamido-7α-méthoxy-3-[1-(2-hydroxy-$C_{2-4}$-alkyl)-1H-tétrazol-5-yl]thiométhyl-1-déthia-1-oxa-3-céphem-4-carbonylique, qui comprend la dissolution d'un glucose, d'un fructose, d'un maltose ou d'un sel de métal alcalin d'un acide minéral ou d'un acide carboxylique comme stabilisant dans un rapport en poids du stabilisant à l'antibiotique de 0,01:1 à 0,5:1 dans un solvant aqueux stérile, la neutralisation de la solution à pH 5—8, la congélation à une température entre −10 et −60°C pendant 2 minutes à 10 heures, et le séchage de la matière congelée sous un vide élevé entre 0,005 et 1 millibar pendant 5 à 72 heures.

2. Procédé suivant la revendication 1, caractérisé en ce que l'antibiotique est le sel de sodium d'acide 7β-difluorométhylthioacétamido-7α-méthoxy-3-[1-(2-hydroxyéthyl)-1H-tétrazol-5-yl]thiométhyl-1-déthia-1-oxa-3-céphen-4-carboxylique.

3. Procédé suivant la revendication 1, caractérisé en ce que le stabilisant est un sel de métal alcalin d'un acide minéral.

4. Procédé suivant la revendication 1, caractérisé en ce que le stabilisant est du bisulfite de sodium, du sulfate de sodium ou du chlorure de sodium.

5. Procédé suivant la revendication 1, caractérisé en ce que le stabilisant est un sel de sodium d'un acide carboxylique dibasique en $C_{3-6}$ choisi parmi l'acide succinique, l'acide fumarique et l'acide maléique.

6. Procédé suivant l'une quelconque des revendications 1 à 5, caractérisé en ce que le rapport en poids du stabilisant à l'antibiotique est de 0,02:1 à 0,3:1.

7. Procédé suivant la revendication 1, caractérisé en ce qu'il est réalisé au moyen d'une lyophilisation en flacon, par vaporisation ou en plateau.

8. Procédé suivant la revendication 7, caractérisé en ce que la préparation lyophilisée est maintenue sous de l'azote sec.